# EUROPEAN PATENT SPECIFICATION

(11) **EP 0 428 169 B1**
(45) Date of publication and mention of the grant of the patent: **01.03.1995**
(21) Application number: 90121888.3
(22) Date of filing: 15.11.1990
(51) Int. Cl.: A61K 31/71, A61K 47/14, A61K 47/28, A61K 47/44

(54) **Aqueous liquid formulations**
Wässrige flüssige Arzneizubereitungen
Compositions aqueuses liquides

(30) Priority: 15.11.1989 GB 8925797
(43) Date of publication of application: 22.05.1991
(73) Proprietor: FUJISAWA PHARMACEUTICAL CO., LTD., Osaka-shi Osaka 541 (JP)
(72) Inventor: Honbo, Toshiyasu, Chuo-ku, Kobe-shi, Hyogo 650 (JP)
(74) Representative: Türk, Gille, Hrabal, Leifert

(56) References cited:
- EP-A- 0 184 162
- EP-A- 0 315 978
- EP-A- 0 323 042
- Fiedler H.P. , "Lexikon der Hilfsstoffe für Pharmazie, Kosmetik und angrenzende Gebiete", 3rd ed., 1989, p. 894, section "Olivenöl"

## Description

This invention relates to new pharmaceutical formulations containing certain known compounds, and to the use of such formulations.

European Patent Application EP-A-0184162 (to Fujisawa Pharmaceutical Co., Ltd.) discloses a number of macrolides isolated from microorganisms belonging to the genus Streptomyces. For example, the macrolides, which are produced by fermentation of Streptomyces tsukubaensis No. 9993 (FERM BP-927) or Streptomyces hygroscopicus subsp. yakushimaensis No. 7238 (FERM BP-928), are numbered FR-900506, FR-900520, FR-900523 and FR-900525. International Patent Application WO-A-89/05304 (to Fisons plc) also discloses a number of macrocyclic compounds. The compounds disclosed in the documents mentioned above, and their Pharmaceutically acceptable salts, were indicated inter alia for use in the treatment of resistance to transplanted organs, autoimmune diseases and infectious diseases.

We have now found a number of novel aqueous liquid formulations suitable for administration of those compounds.

Thus, according to the present invention, there are provided aqueous liquid formulations comprising, as an active ingredient, at least one compound of formula I, 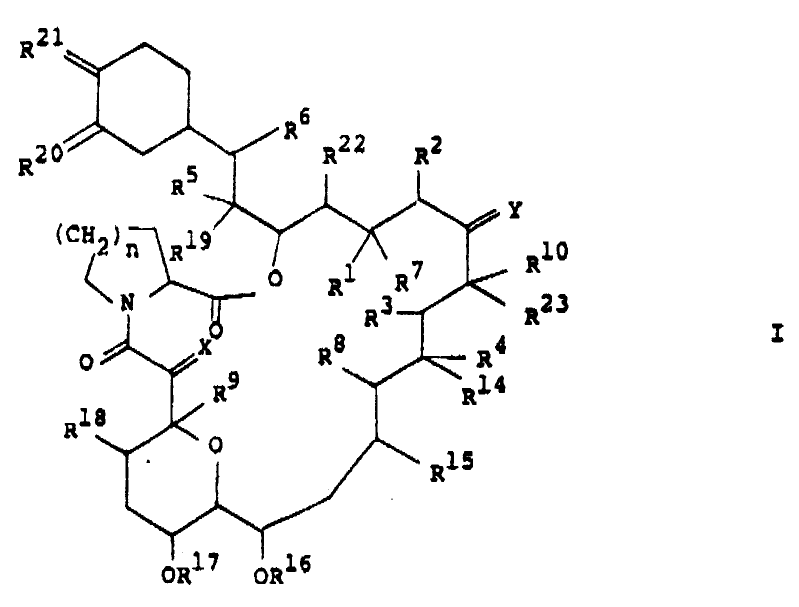
wherein each vicinal pair of substituents [R¹ and R²],
[R³ and R⁴], [R⁵ and R⁶] independently
a) represent two vicinal hydrogen atoms, or
b) form a second bond between the vicinal carbon atoms to which they are attached;
in addition to its significance above, R² may represent an alkyl group;
- R⁷: represents H, OH, protected hydroxy or O-alkyl, or in conjunction with R¹ it may represent =O;
- R⁸ and R⁹: independently represent H or OH;
- R¹⁰: represents H, alkyl, alkyl substituted by one or more hydroxyl groups, alkenyl, alkenyl substituted by one or more hydroxyl groups, or alkyl substituted by =O;
- X: represents O, (H,OH), (H,H) or -CH₂O-;
- Y: represents O, (H,OH), (H,H), N-NR¹¹R¹² or N-OR¹³;
- R¹¹ and R¹²: independently represent H, alkyl, aryl or tosyl;
- R¹³, R¹⁴, R¹⁵, R¹⁶, R¹⁷, R¹⁸, R¹⁹, R²² and R²³: independently represent H or alkyl;
- R²⁰ and R²¹: independently represent O, or they may independently represent (R²⁰a,H) and (R²¹a,H) respectively; R²⁰a and R²¹a independently represent OH, O-alkyl or OCH₂OCH₂CH₂OCH₃ or R²¹a is protected hydroxy;
in addition, R²⁰a and R²¹a may together represent an oxygen atom in an epoxide ring;
n is 1, 2 or 3;
in addition to their significances above, Y, R¹⁰ and R²³, together with the carbon atoms to which they are attached, may represent a 5- or 6-membered N-, S- or O- containing heterocyclic ring, which may be saturated or unsaturated, and which may be substituted by one or more groups selected from alkyl, hydroxy, alkyl substituted by one or more hydroxyl groups, O-alkyl, benzyl and -CH₂Se(C₆H₅),
or a pharmaceutically acceptable salt thereof, in admixture with at least one surfactant selected from the group consisting of sodium taurocholate, esters or partial esters of fatty acids, the oleates of sorbitan and polyoxyethylated hydrogenated caster oils.

The specific examples of the definitions of compound (I) and the preferred working modes of the invention are described in detail below.

The term " lower " as used in this specification means, unless otherwise indicated, any number of carbon atoms between 1 and 6, inclusive.

Suitable " alkyl ", means straight or branched saturated aliphatic hydrocarbon residue and may include lower alkyl such as methyl, ethyl, propyl, isopropyl, butyl, isobutyl, pentyl, neopentyl, hexyl, and the like.

Suitable " alkenyl " means straight or branched unsaturated aliphatic hydrocarbon residue having one double bond and may include lower alkenyl such as vinyl, propenyl, butenyl, methylpropenyl, pentenyl, hexenyl, and the like.

Suitable " aryl " may include phenyl, tolyl, xylyl, cumenyl, mesityl, naphthyl, and the like.

Suitable examples of the protective group in the " protected hydroxyl group " may include:
1-(lower alkylthio)(lower)alkyl groups such as lower alkylthiomethyl groups (e.g. methylthiomethyl, ethylthiomethyl, propylthiomethyl, isopropylthiomethyl, butylthiomethyl, isobutylthiomethyl, hexylthiomethyl, etc.), more desirably C₁-C₄ alkylthiomethyl groups, and most desirably methylthiomethyl;
tri-substituted silyl groups such as tri(lower)alkylsilyl groups (e.g. trimethylsilyl, triethylsilyl, tributylsilyl, tert-butyl-dimethylsilyl, tri-tert-butylsilyl, etc.);
lower alkyl-diarylsilyl groups (e.g. methyldiphenylsilyl, ethyldiphenylsilyl, propyldiphenylsilyl, tert-butyldiphenylsilyl, etc.), more desirably tri(C₁-C₄)alkylsilyl and C₁-C₄ alkyldiphenylsilyl groups and most desirably tert-butyldimethylsilyl and tert-butyldiphenylsilyl; and acyl groups such as aliphatic acyl groups, aromatic acyl groups and aliphatic acyl groups substituted by aromatic groups, which are derived from carboxylic acids, sulfonic acids or carbamic acids.

The aliphatic acyl group may includes lower alkanoyl groups which may optionally have one or more suitable substituents such as carboxy (e.g. formyl, acetyl, propionyl, butyryl, isobutyryl, valeryl, isovaleryl, pivaloyl, hexanoyl, carboxyacetyl, carboxypropionyl, carboxybutyryl, carboxyhexanoyl, etc.), cyclo(lower)alkoxy(lower)alkanoyl groups which may optionally have one or more appropriate substituents such as lower alkyl (e.g. cyclopropyloxyacetyl, cyclobutyloxypropionyl, cycloheptyloxybutyryl, menthyloxyacetyl, menthyloxypropionyl, menthyloxybutyryl, menthyloxypentanoyl, menthyloxyhexanoyl, etc.), camphorsulfonyl, lower alkylcarbamoyl groups having one or more suitable substituents such as carboxy or protected carboxy, for example carboxy(lower)alkylcarbamoyl groups( e.g. carboxymethylcarbamoyl, carboxyethylcarbamoyl, carboxypropylcarbamoyl, carboxybutylcarbamoyl, carboxypentylcarbamoyl, carboxyhexylcarbamoyl, etc.) protected carboxy(lower)alkylcarbamoyl groups such as tri(lower)alkylsilyl(lower)alkoxycarbonyl(lower)alkylcarbamoyl groups(e.g. trimethylsilylmethoxycarbonylethylcarbamoyl, trimethylsilylethoxycarbonylpropyl carbamoyl, triethylsilylethoxycarbonylpropylcarbamoyl, tert-butyldimethylsilylethoxycarbonylpropylcarbamoyl, trimethylsilylpropoxycarbonylbutylcarbamoyl, etc.) and so on.

The aromatic acyl group may include aroyl groups which may optionally have one or more suitable substituents such as nitro (e.g. benzoyl, toluoyl, xyloyl, naphthoyl, nitrobenzoyl, dinitrobenzoyl, nitronaphthoyl, etc), arenesulfonyl groups which may optionally have one or more suitable substituent(s) such as halogen (e.g. benzenesulfonyl, toluenesulfonyl, xylenesulfonyl, naphthalenesulfonyl, fluorobenzenesulfonyl, chlorobenzenesulfonyl, bromobenzenesulfonyl, iodobenzenesulfonyl, etc.), and so on.

The aromatic group substituted aliphatic acyl group may include ar(lower)alkanoyl groups which may optionally have one or more suitable substituent(s) such as lower alkoxy and trihalo(lower)alkyl (e.g. phenylacetyl, phenylpropionyl, phenylbutyryl, 2-trifluoromethyl-2-methoxy-2-phenylacetyl, 2-ethyl-2-trifluoromethyl-2-phenylacetyl, 2-trifluoromethyl-2-propoxy-2-phenylacetyl, etc.), and so on.

Among the above-mentioned acyl groups, the more desirable acyl groups are C₁-C₄ alkanoyl groups which may optionally be substituted by carboxy, cyclo(C₅-C₆)alkyloxy(C₁-C₄)alkanoyl groups having two (C₁-C₄)alkyl groups in the cycloalkyl moiety, camphorsulfonyl, carboxy(C₁-C₄)alkylcarbamoyl groups, tri(C₁-C₄)alkylsilyl(C₁-C₄)alkoxycarbonyl(C₁-C₄)alkylcarbamoyl groups, benzoyl which may have one or two nitro groups, halogen-substituted benzenesulfonyl groups, phenyl(C₁-C₄)alkanoyl groups having C₁-C₄ alkoxy and trihalo(C₁-C₄)alkyl groups. Of these groups, the most desirable are acetyl, carboxypropionyl, menthyloxyacetyl, camphorsulfonyl, benzoyl, nitrobenzoyl, dinitrobenzoyl, iodobenzenesulfonyl and 2-trifluoromethyl-2-methoxy-2-phenylacetyl.

Suitable " 5- or 6-membered N-, S- or O-containing heterocyclic ring " may include pyrrolyl, tetrahydrofuryl, and the like.

The pharmaceutically acceptable salt of compound (I) is a nontoxic salt, which may be the corresponding salt with an inorganic or organic base such as alkali metal salts (e.g. sodium salt, potassium salt, etc.), alkaline earth metal salts (e.g. calcium salt, magnesium salt, etc.), ammonium salt and amine salts (e.g. triethylamine salt, N-benzyl-N-methylamine salt, etc.) and so on.

Referring to compound (I), there may exist conformers or one pair or more of stereoisomers such as optical and geometrical isomers due to the asymmetric carbon or the double bond. Such conformers and isomers also fall within the scope of the invention.

One of the suitable surfactants is sodium taurocholate, an anionic surfactant. However, we prefer the surfactant to be non-ionic, that is, we prefer the esters or partial esters of fatty acids containing from 6 to 22 carbon atoms, such as caproic, octoic, lauric, palmitic, stearic, linoleic, linolenic, oleostearic and oleic acids with an aliphatic polyhydric alcohol or its cyclic anhydride such as, for example, ethylene glycol, glycerol, erythritol, arabitol, mannitol, sorbitol, the hexitol anhydrides derived from sorbitol (the sorbitan esters sold under the Registered Trade Mark "SPAN") and the polyoxyethylene and polyoxypropylene derivatives of these esters. Mixed esters, such as mixed or natural glycerides, may be employed. Further non-ionic surfactants which may be mentioned are the oleates of sorbitan, eg those sold under the Registered Trade Marks "Arlacel C" (sorbitan sesquioleate), "Span 80" (sorbitan monooleate) and "Span 85" (Sorbitan trioleate). Specific examples of other non-ionic surfactants which may be employed are sorbitan monolaurate, polyoxyethylene sorbitol tetraoleate, polyoxyethylene sorbitol pentaoleate and polyoxypropylene mannitol dioleate. Where available, the hydrogenated, ethoxylated, polyoxyethylated, or hydrogenated polyoxyethylated derivatives of the above-mentioned surfactants may be employed.

The preferred non-ionic surfactants are polyoxyethylated hydrogenated castor oils, such as HCO-20, HCO-40 and HCO-60 (Registered Trade Marks).

The weight ratio of surfactant to the compound (I) is not limited, preferably 1∼100 mg: 1 mg and more preferably 30∼60 mg: 1 mg.

The mode of administration of the formulations of the present invention should not be limited. Preferably they may be administered externally, enterally or parenterally (e.g. intravenously, etc).

For the above-mentioned therapeutic uses the dosage administered will, of course, vary with the compound employed, the mode of administration, the treatment desired (e.g. parenteral or oral) and the disease indicated. In the case of treatment of resistance to transplanted organs, for example a transplanted liver, suitable dose regimes would be administration of from 0.01 to 1 mg.kg⁻¹ of body weight, preferably from 0.075 to 0.5 mg.kg⁻¹, of active ingredient to a patient intravenously every 12 hours following surgery. This may then be followed by oral administration of from 0.1 to 10 mg.kg⁻¹ of body weight, preferably from 0.15 to 5 mg.kg⁻¹, of active ingredient when the patient is able to absorb the drug from the gastro-intestinal tract (usually 3 days following surgery), at an initial frequency of every 12 hours, decreasing gradually to a frequency of every 3 days for as long as therapy needs to continue.

The dose regimes described above forms a second aspect of the present invention.

The invention is illustrated but in no way limited by the following examples.

### Example

| | |
|---|---|
| FR-900506 | 12.5 mg |
| Polyoxyethylated (60 mol) hydrogenated caster oil (HCO-60) | 100.0 mg |
| Glyceryl monooleate | 30.0 mg |
| Sodium taurocholate | 40.0 mg |
| D-Mannitol | 66.5 mg |
| Distilled water | |
| | to 1 ml |

The D-mannitol was dissolved in the distilled water, and the polyoxyethylated hydrogenated castor oil, glyceryl monooleate and sodium taurocholate were then added, followed by FR-900506. Stirring was continued until solution was complete. The formulation thus prepared can be administered orally.

## Claims

1. An aqueous livid formulation which comprises at least one compound of the formula : 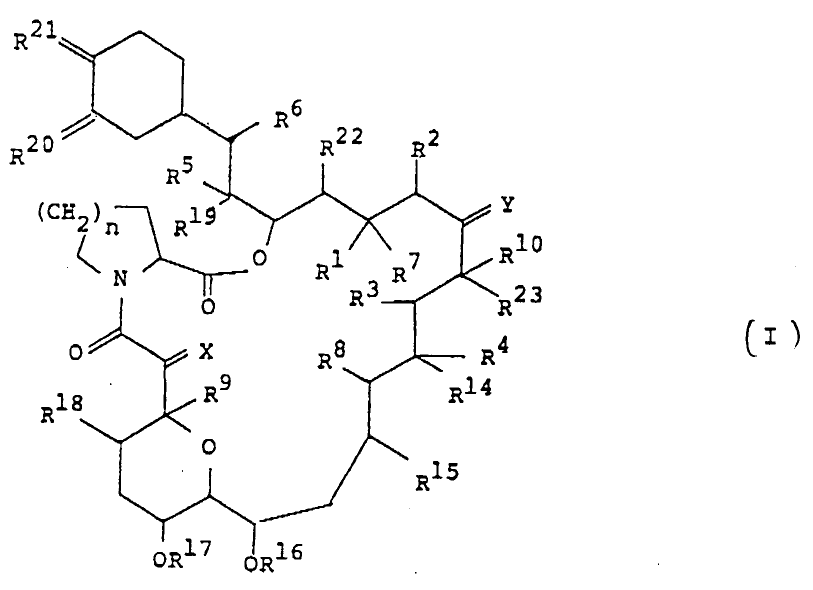 wherein each vicinal pair of substituents [R¹ and
R²], [R³ and R⁴], [R⁵ and R⁶] independently
a) represent two vicinal hydrogen atoms, or
b) form a second bond between the vicinal carbon atoms to which they are attached;
in addition to its significance above, R² may represent an alkyl group;
R⁷ represents H, OH, protected hydroxy or O-alkyl, or in conjunction with R¹ it may represent =O;
R⁸ and R⁹ independently represent H or OH;
R¹⁰ represents H, alkyl, alkyl substituted by one or more hydroxyl groups, alkenyl, alkenyl substituted by one or more hydroxyl groups, or alkyl substituted by =O;
X represents O, (H,OH), (H,H) or -CH₂O-;
Y represents O, (H,OH), (H,H), N-NR¹¹R¹² or N-OR¹³;
R¹¹ and R¹² independently represent H, alkyl, aryl or tosyl;
R¹³, R¹⁴, R¹⁵, R¹⁶, R¹⁷, R¹⁸, R¹⁹, R²² and R²³ independently represent H or alkyl;
R²⁰ and R²¹ independently represent O, or they may independently represent (R²⁰a,H) and (R²¹a,H) respectively; R²⁰a and R²¹a independently represent OH, O-alkyl or OCH₂OCH₂CH₂OCH₃ or R²¹a is protected hydroxy;
in addition, R²⁰a and R²¹a may together represent an oxygen atom in an epoxide ring;
n is 1, 2 or 3;
in addition to their significances above, Y, R¹⁰ and R²³, together with the carbon atoms to which they are attached, may represent a 5- or 6-membered N-, S- or O- containing heterocyclic ring, which may be saturated or unsaturated, and which may be substituted by one or more groups selected from alkyl, hydroxy, alkyl substituted by one or more hydroxyl groups, O-alkyl, benzyl and -CH₂Se(C₆H₅),
or a pharmaceutically acceptable salt thereof and at least one surfactant selected from the group consisting of sodium taurocholate, esters or partial esters of fatty acids, the oleates of sorbitan and polyoxyethylated hydrogenated castor oils.

2. The aqueous liquid formulation of claim 1, in which the compound (I) is 17-allyl-1,14-dihydroxy-12-[2-(4-hydroxy-3-methoxcyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone.

3. A process for preparing an
aqueous liquid formulation which comprises admixing at least one compound of the formula : 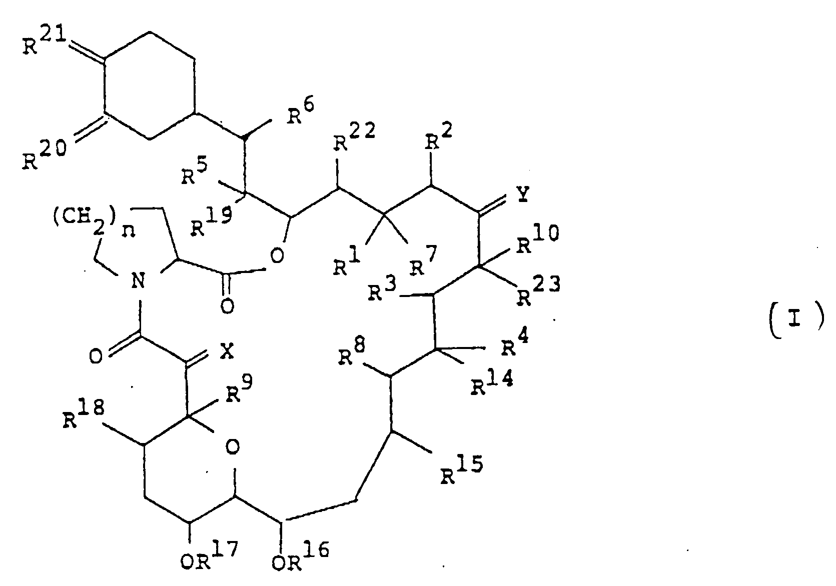 wherein each vicinal pair of substituents [R¹ and
R²], [R³ and R⁴], [R⁵ and R⁶] independently
a) represent two vicinal hydrogen atoms, or
b) form a second bond between the vicinal carbon atoms to which they are attached;
in addition to its significance above, R² may represent an alkyl group;
R⁷ represents H, OH, protected hydroxy or O-alkyl, or in conjunction with R¹ it may represent =O;
R⁸ and R⁹ independently represent H or OH;
R¹⁰ represents H, alkyl, alkyl substituted by one or more hydroxyl groups, alkenyl, alkenyl substituted by one or more hydroxyl groups, or alkyl substituted by =O;
X represents O, (H,OH), (H,H) or -CH₂O-;
Y represents O, (H,OH), (H,H), N-NR¹¹R¹² or N-OR¹³;
R¹¹ and R¹² independently represent H, alkyl, aryl or tosyl;
R¹³, R¹⁴, R¹⁵, R¹⁶, R¹⁷ R¹⁸, R¹⁹, R²² and R²³ independently represent H or alkyl;
R²⁰ and R²¹ independently represent O, or they may independently represent (R²⁰a,H) and (R²¹a,H) respectively; R²⁰a and R²¹a independently represent OH, O-alkyl or OCH₂OCH₂CH₂OCH₃ or R²¹a is protected hydroxy;
in addition, R²⁰a and R²¹a may together represent an oxygen atom in an epoxide ring;
n is 1, 2 or 3;
in addition to their significances above, Y, R¹⁰ and R²³, together with the carbon atoms to which they are attached, may represent a 5- or 6-membered N-, S- or O- containing heterocyclic ring, which may be saturated or unsaturated, and which may be substituted by one or more groups selected from alkyl, hydroxy, alkyl substituted by one or more hydroxyl groups, O-alkyl, benzyl and -CH₂Se(C₆H₅),
or a pharmaceutically acceptable salt thereof with at least one surfactant selected from the group consisting of sodium taurocholate, esters or partial esters of fatty acids, the oleates of sorbitan and polyoxyethylated hydrogenated castor oils.

4. The process of claim 3,
in which the compound (I) is 17-allyl-1,14-dihydroxy-12-[2-(4-hydroxy-3 methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone.

## Patentansprüche

1. Eine wäßrige flüssige Formulierung, die mindestens eine Verbindung der Formel umfaßt: 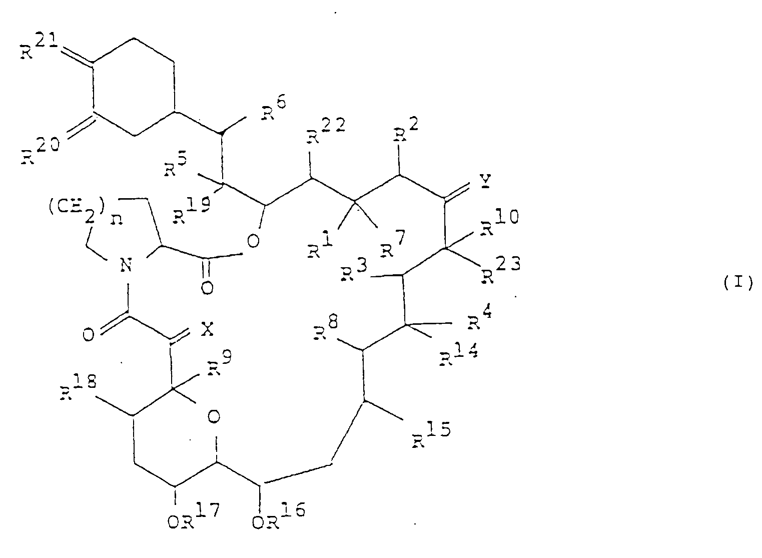 worin jedes vicinale Substituentenpaar [R¹ und R²], [R³ und R⁴], [R⁵ und R⁶] unabhängig
a) zwei vicinale Wasserstoffatome darstellen, oder
b) eine zweite Bindung zwischen den vicinalen Kohlenstoffatomen bilden, an die sie gebunden sind,
R² zusätzlich zu seiner obigen Bedeutung eine Alkylgruppe darstellen kann;
R⁷ H, OH, geschütztes Hydroxy oder O-Alkyl darstellt oder in Verbindung mit R¹ =O darstellen kann;
R⁸ und R⁹ unabhängig H oder OH darstellen,
R¹⁰ H, Alkyl, durch eine oder mehrere Hydroxygruppen substituiertes Alkyl, Alkenyl, durch eine oder mehrere Hydroxygruppen substituiertes Alkenyl oder durch =O substituiertes Alkyl darstellt;
X O, (H,OH), (H,H) oder -CH₂O- darstellt;
Y O, (H,OH), (H,H), N-NR¹¹R¹² oder N-OR¹³ darstellt;
R¹¹ und R¹² unabhängig H, Alkyl, Aryl oder Tosyl darstellen,
R¹³, R¹⁴, R¹⁵, R¹⁶, R¹⁷, R¹⁸, R¹⁹, R²² und R²³ unabhängig H oder Alkyl darstellen;
R²⁰ und R²¹ unahängig O darstellen, oder unabhängig (R²⁰a,H) und R²¹a,H) darstellen können; R²⁰a und R²¹a unabhängig OH, O-Alkyl oder OCH₂OCH₂CH₂OCH₃ darstellen oder R²¹a geschütztes Hydroxy ist;
R²⁰a und R²¹a zusätzlich gemeinsam ein Sauerstoffatom in einem Epoxidring darstellen können;
n 1, 2 oder 3 ist;
Y, R¹⁰ und R²³ zusätzlich zu ihren obigen Bedeutungen gemeinsam mit den Kohlenstoffatomen, an die sie gebunden sind, einen 5- oder 6-gliedrigen N-, S- oder O- enthaltenden heterocyclischen Ring darstellen können, der gesättigt oder ungesättigt sein kann, und der durch eine oder mehr aus Alkyl, Hydroxy, durch eine oder mehr Hydroxygruppen substituiertes Alkyl, O-Alkyl, Benzyl und -CH₂Se(C₆H₅) ausgewählte Gruppen substituiert sein kann,
oder ein pharmazeutisch verträgliches Salz davon, und mindestens ein oberflächenaktives Mittel, ausgewählt aus der aus Natriumtaurocholat, Estern oder partiellen Estern von Fettsäuren, den Oleaten von Sorbitan und polyoxyethylierten hydrogenierten Castorölen bestehenden Gruppe.

2. Die wäßrige flüssige Formulierung aus Anspruch 1, in der die Verbindung (I) 17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon ist.

3. Ein Verfahren zur Herstellung einer wäßrigen flüssigen Formulierung, welches umfaßt: Mischen mindestens einer Verbindung der Formel: 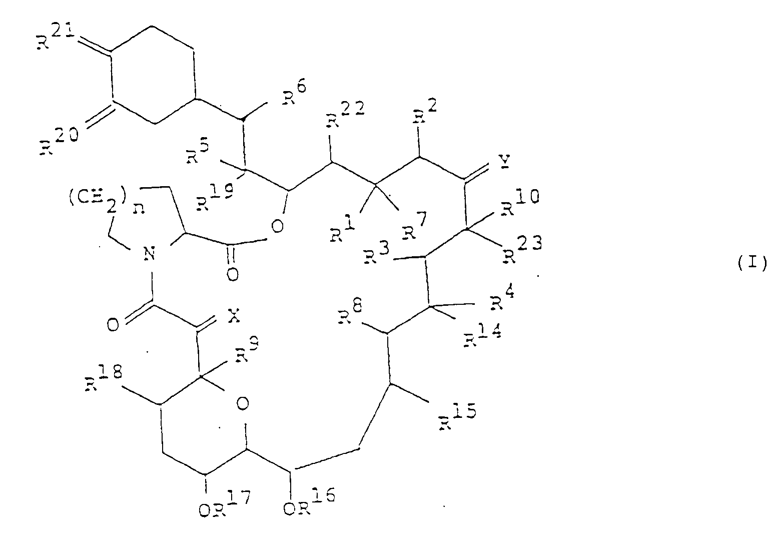 worin jedes vicinale Substituentenpaar [R¹ und R²], [R³ und R⁴), [R⁵ und R⁶] unabhängig
a) zwei vicinale Wasserstoffatome darstellen, oder
b) eine zweite Bindung zwischen den vicinalen Kohlenstoffatomen bilden, an die sie gebunden sind,
R² zusätzlich zu seiner obigen Bedeutung eine Alkylgruppe darstellen kann;
R⁷ H, OH, geschütztes Hydroxy oder O-Alkyl darstellt oder in Verbindung mit R¹ =O darstellen kann;
R⁸ und R⁹ unabhängig H oder OH darstellen,
R¹⁰ H, Alkyl, durch eine oder mehrere Hydroxygruppen substituiertes Alkyl, Alkenyl, durch eine oder mehrere Hydroxygruppen substituiertes Alkenyl oder durch =O substituiertes Alkyl darstellt;
X O, (H,OH), (H,H) oder -CH₂O- darstellt;
Y O, (H,OH), (H,H), N-NR¹¹R¹² oder N-OR¹³ darstellt;
R¹¹ und R¹² unabhängig H, Alkyl, Aryl oder Tosyl darstellen,
R¹³, R¹⁴, R¹⁵, R¹⁶, R¹⁷, R¹⁸, R¹⁹, R²² und R²³ unabhängig H oder Alkyl darstellen;
R²⁰ und R²¹ unahängig O darstellen, oder unabhängig (R²⁰a,H) und R²¹a,H) darstellen können; R²⁰a und R²¹a unabhängig OH, O-Alkyl oder OCH₂OCH₂CH₂OCH₃ darstellen oder R²¹a geschütztes Hydroxy ist;
R²⁰a und R²¹a zusätzlich gemeinsam ein Sauerstoffatom in einem Epoxidring darstellen können;
n 1, 2 oder 3 ist;
Y, R¹⁰ und R²³ zusätzlich zu ihren obigen Bedeutungen gemeinsam mit den Kohlenstoffatomen, an die sie gebunden sind, einen 5- oder 6-gliedrigen N-, S- oder O- enthaltenden heterocyclischen Ring darstellen können, der gesättigt oder ungesättigt sein kann, und der durch eine oder mehr aus Alkyl, Hydroxy, durch eine oder mehr Hydroxygruppen substituiertes Alkyl, O-Alkyl, Benzyl und -CH₂Se(C₆H₅) ausgewählte Gruppen substituiert sein kann,
oder eines pharmazeutisch verträgliches Salzes davon mit mindestens einem oberflächenaktiven Mittel, ausgewählt aus der aus Natriumtaurocholat, Estern oder partiellen Estern von Fettsäuren, den Oleaten von Sorbitan und polyoxyethylierten hydrogenierten Castorölen bestehenden Gruppe.

4. Das Verfahren von anspruch 3, in dem die Verbindung (I) 17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo(22.3.1.0^{4,9}]octacos-18-en-2,3,10,19-tetraon ist.

## Revendications

1. Composition liquide aqueuse qui comprend au moins un composé de formule : 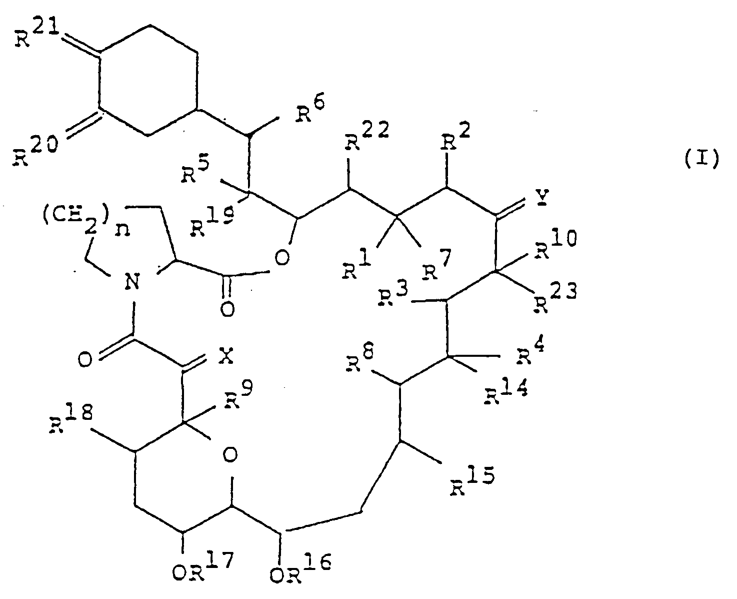 dans laquelle chaque paire contiguë de substituants [R¹ et R²], [R³ et R⁴], [R⁵ et R⁶] indépendamment
a) représentent deux atomes d'hydrogène contigus, ou
b) forment une seconde liaison entre les atomes contigus de carbone auxquels ils sont liés,
en plus de sa signification ci-dessus, R² peut représenter un groupe alkyle;
R⁷ représente H, OH, un hydroxy protégé ou O-alkyle, ou conjointement avec R¹ il peut représenter =O;
R⁸ et R⁹ indépendamment représentent H ou OH;
R¹⁰ représente H, un alkyle, un alkyle substitué avec un ou plusieurs groupes hydroxy, un alcényle, un alcényle substitué avec un ou plusieurs groupes hydroxy, ou un alkyle substitué avec =O;
X représente O, (H,OH), (H,H) ou -CH₂O-;
Y représente O, (H,OH), (H,H), N-NR¹¹R¹² ou N-OR¹³,
R¹¹ et R¹² indépendamment représentent H, un alkyle, un aryle ou un tosyle,
R¹³, R¹⁴, R¹⁵, R¹⁶, R¹⁷, R¹⁸, R¹⁹, R²² et R²³ indépendamment représentent H ou un alkyle;
R²⁰ et R²¹ indépendamment représentent O, ou ils peuvent indépendamment représenter (R²⁰a,H) et (R²¹a,H) respectivement; R²⁰a et R²¹a indépendamment représentent OH, O-alkyle ou OCH₂OCH₂CH₂OCH₃ ou R²¹a est un hydroxy protégé;
en outre, R²⁰a et R²¹a peuvent ensemble représenter
un atome d'oxygène dans un cycle époxyde,
n est égal à 1, 2, ou 3,
en plus de leurs significations précédentes, Y, R¹⁰ et R²³, ensemble avec les atomes de carbone auxquels ils sont liés, peuvent représenter un groupe hétérocyclique à 5 ou 6 membres contenant N, S ou O, qui peut être saturé ou insaturé, et qui peut être substitué avec un ou plusieurs groupes choisis parmi un alkyle, un hydroxy, un alkyle substitué avec un ou plusieurs groupes hydroxy, O-alkyle, un benzyle et -CH₂Se(C₆H₅);
ou un sel pharmaceutiquement acceptable de celui-ci, et au moins un agent tensioactif choisi dans le groupe constitué par le taurocholate de sodium, les esters ou esters partiels d'acides gras, les oléates de sorbitane et les huiles de ricin hydrogénées polyoxyéthylées.

2. Composition liquide aqueuse selon la revendication 1, dans laquelle le composé (I) est la 17-allyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone.

3. Procédé pour la préparation d'une composition liquide aqueuse qui comprend le mélange d'un composé de formule : 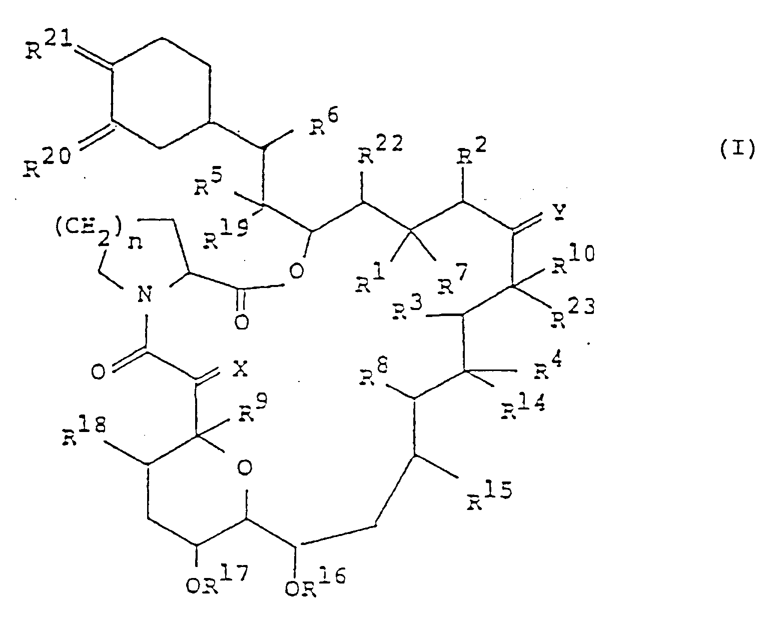 dans laquelle chaque paire contiguë de substituant [R¹ et R²], [R³ et R⁴], [R⁵ et R⁶] indépendamment
a) représentent deux atomes d'hydrogène contigus, ou
b) forment une seconde liaison entre les atomes contigus de carbone auxquels ils sont liés,
en plus de sa signification ci-dessus, R² peut représenter un groupe alkyle;
R⁷ représente H, OH, un hydroxy protégé ou O-alkyle, ou conjointement avec R¹ il peut représenter =O;
R⁸ et R⁹ indépendamment représentent H ou OH;
R¹⁰ représente H, un alkyle, un alkyle substitué avec un ou plusieurs groupes hydroxy, un alcényle, un alcényle substitué avec un ou plusieurs groupes hydroxy, ou un alkyle substitué avec =O;
X représente O, (H,OH), (H,H) ou -CH₂O-;
Y représente O, (H,OH), (H,H), N-NR¹¹R¹² ou N-OR¹³,
R¹¹ et R¹² indépendamment représentent H, un alkyle, un aryle ou un tosyle,
R¹³, R¹⁴, R¹⁵, R¹⁶, R¹⁷, R¹⁸, R¹⁹, R²² et R²³ indépendamment représentent H ou un alkyle;
R²⁰ et R²¹ indépendamment représentent O, ou ils peuvent indépendamment représenter (R²⁰a,H) et (R²¹a,H) respectivement; R²⁰a et R²¹a indépendamment représentent OH, O-alkyle ou OCH₂OCH₂CH₂OCH₃ ou R²¹a est un hydroxy protégé;
en outre, R²⁰a et R²¹a peuvent ensemble représenter un
atome d'oxygène dans un cycle époxyde,
n est égal à 1, 2, ou 3,
en plus de leurs significations précédentes, Y, R¹⁰ et R²³, ensemble avec les atomes de carbone auxquels ils sont liés, peuvent représenter un groupe hétérocyclique à 5 ou 6 membres contenant N, S ou O, qui peut être saturé ou insaturé, et qui peut être substitué avec un ou plusieurs groupes choisis parmi un alkyle, un hydroxy, un alkyle substitué avec un ou plusieurs groupes hydroxy, O-alkyle, un benzyle et -CH₂Se(C₆H₅);
ou d'un sel pharmaceutiquement acceptable de celui-ci, avec au moins un agent tensioactif choisi dans le groupe constitué par le taurocholate de sodium, les esters ou esters partiels d'acides gras, les oléates de sorbitane et les huiles de ricin hydrogénées polyoxyéthylées.

4. Procédé selon la revendication 3, dans lequel le composé (I) est la 17-allyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2_{,}3,10,16-tétraone.
